(19) Europäisches Patentamt
European Patent Office
Office européen des brevets

(11) Veröffentlichungsnummer : **0 406 162 B1**

(12) # EUROPÄISCHE PATENTSCHRIFT

(45) Veröffentlichungstag der Patentschrift : **08.09.93 Patentblatt 93/36**

(51) Int. Cl.$^5$ : **A61K 9/107,** B01F 17/00, C12N 5/00

(21) Anmeldenummer : **90810436.7**

(22) Anmeldetag : **15.06.90**

(54) **Verfahren zur Herstellung einer Nanoemulsion von Oelpartikeln in einer wässrigen Phase.**

Die Akte enthält technische Angaben, die nach dem Eingang der Anmeldung eingereicht wurden und die nicht in dieser Patentschrift enthalten sind.

(30) Priorität : **26.06.89 CH 2374/89**

(43) Veröffentlichungstag der Anmeldung : **02.01.91 Patentblatt 91/01**

(45) Bekanntmachung des Hinweises auf die Patenterteilung : **08.09.93 Patentblatt 93/36**

(84) Benannte Vertragsstaaten : **AT BE CH DE FR GB IT LI NL SE**

(56) Entgegenhaltungen :
**WO-A-88/08301**
**DE-A- 1 249 454**
**DE-C- 2 938 807**

(73) Patentinhaber : **Weder, Hans Georg, Prof. Dr.**
**Säumerstrasse 68**
**CH-8800 Thalwil (CH)**

(72) Erfinder : **Weder, Hans Georg, Prof. Dr.**
**Säumerstrasse 68**
**CH-8800 Thalwil (CH)**
Erfinder : **Mütsch, Matthias**
**Imbisbühlstrasse 135**
**CH-8049 Zürich (CH)**

(74) Vertreter : **Ryffel, Rolf**
**Hepp, Wenger & Ryffel AG Bahnhofstrasse 58**
**CH-8001 Zürich (CH)**

EP 0 406 162 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezieht sich auf ein Verfahren zur Herstellung einer Nanoemulsion von weniger als 2oo nm grossen Oelpartikeln aus Triglycerid oder Fettsäureester in einer wässrigen Phase mittels eines Hochdruckhomogenisators.

Eine solche Nanoemulsion kann als kolloid-hochdisperses Zweiphasen-System charakterisiert werden. Aufgrund von Laser-Streulichtmessungen und elektronen-mikroskopischen Aufnahmen kann sie klar unterschieden werden von Assoziaten (Mizellen) und Solubilisaten (mizellare Lösungen = gequollene Mizellen), wie sie z.B. beschrieben sind in P.H. List, B.W. Müller und E. Nürnberg, "Emulsionen", in "Arzneiformenlehre", 1982, Wissenschaftliche Verlagsgesellschaft mbH, Stuttgart, Seiten 141-176. Durch die Partikelgrösse von weniger als 200 nm grenzt sich die Nanoemulsion von grob dispersen Systemen ab.

In H. Sucker, P. Fuchs und P. Speiser, "Dermatica", in "Pharmazeutische Technologie", 1978, Georg Thieme Verlag, Stuttgart, Seiten 663-665, sind sog. "Mikroemulsionen" beschrieben. Da die Bedeutung des Begriffes "Mikroemulsion" jedoch bis heute nicht eindeutig geklärt ist, wird hier zur Bezeichnung einer Emulsion mit weniger als 200 nm grossen Oelpartikeln der Begriff Nanoemulsion verwendet.

Bei der bekannten Herstellung von "Mikroemulsionen" sind hochkonzentrierte Tensid-Kotensid-Gemische erforderlich, um eine minimale Oberflächenspannung zu bewirken und die physikalische Stabilität der Emulsion zu gewährleisten. Die grössten Nachteile aus pharmazeutischer Sicht sind die hohen Tensid-Kotensid-Konzentrationen, die zur Herstellung notwendig sind, und die Toxizität der bekannten Emulgatorkomplexe. Eine praktische Anwendung der "Mikroemulsionen" z.B. als Arzneistoffträger für lipophile Wirkstoffe oder ihr Einsatz zur parenteralen Ernährung konnte wegen der erwähnten Nachteile bisher nicht ins Auge gefasst werden.

In DE-C-29 38 807 ist eine Emulsion von Partikeln aus Sojabohnenoel mit einer mittleren Grösse von höchstens 100 nm beschrieben. Als Emulgator werden Eigelbphospholipide verwendet. Zusätzlich sind für die Erreichung der angegebenen kleinen Partikelgrösse eine Fettsäure oder Fettsäuresalz und Cholesterin oder Cholesterinderivat als Emulgierhilfen erforderlich. Für die Herstellung der Emulsion werden die Eigelbphospholipide und die Emulgierhilfen im Sojabohnenoel gelöst. Anschliessend wird Wasser zugesetzt, durchmischt und in einem Hochdruckhomogenisator fein emulgiert. Zu Vergleichszwecken wird in diesem Dokument auch die Herstellung von Emulsionen in gleicher Weise, jedoch ohne Verwendung von zusätzlichen Emulgierhilfen beschrieben. Dazu ist angegeben, dass in dieser Weise Emulsionen mit Partikelgrössen von 150 nm erhalten werden können.

Die Aufgabe der Erfindung besteht darin, ein Verfahren der eingangs angegebenen Art zur Verfügung zu stellen, mit welchem ohne Verwendung von hochkonzentrierten Tensid-Kotensid-Komplexen oder anderen Emulgierhilfen stabile Nanoemulsionen hergestellt werden können, die sich durch Dampfdrucksterilisation oder Keimfiltration antimikrobiell behandeln lassen und die, zumindest während mehrerer Monate, stabil bleiben.

Das erfindungsgemässe Verfahren, mit dem die Aufgabe gelöst wird, ist dadurch gekennzeichnet, dass die wässrige Phase, das Oel und pro Gewichtsteil Oel 0,05 bis 0,4 Gewichtsteile eines amphoteren Glycerinphosphatid-Emulgators so zusammengemischt werden, dass der Emulgator in der wässrigen Phase eine Doppelschicht-Struktur bildet, und dass die Mischung dann in dem Hochdruckhomogenisator zur Nanoemulsion verarbeitet wird.

Die Doppelschicht-Struktur des Emulgators entspricht einer lamellaren flüssig-kristallinen Struktur, die dann in der Emulsion die Grenzschicht zwischen der äusseren wässrigen Phase und der inneren Oelphase bildet. In der nachfolgenden Beschreibung wird der Ausdruck "lamellare flüssig-kristalline Struktur" verwendet.

In DE-B-1 249 454, wo ebenfalls Verfahren zur Herstellung von Emulsionen von Sojabohnenoel mit Eiphosphatiden als Emulgator beschrieben werden, sind zwar Emulgierhilfen nur als fakultative Bestandteile erwähnt. Die Emulsionen haben jedoch wesentlich grössere Partikel als die erfindungsgemäss hergestellten Emulsionen, nämlich Partikel von bis zu 4000 nm oder vorzugsweise bis zu 1000 nm. Das Sojabohnenoel wird einfach mit Eiphosphatiden und Wasser zusammengemischt, ohne dass die Eiphosphatide eine lamellare flüssig-kristalline Struktur bilden könnten.

Mit dem erfindungsgemässen Verfahren können reproduzierbare, zumindest während mehrerer Monate stabile Nanoemulsionen hergestellt werden, die toxikologisch unbedenklich sind und die daher als Arzneistoffträger, in Parenteralien, in kosmetischen Präparaten und in Nährstofflösungen (Zellkulturmedien für die Biotechnologie) verwendet werden können. Eine solche Nanoemulsion kann auch als Träger für einen Stoff mit sauerstoffübertragender Funktion verwendet werden und damit als Blutersatz dienen.

Ein solcher Stoff oder Sauerstoffträger, der molekularen Sauerstoff binden kann, ist z.B. mit Sauerstoff gesättigtes Hämoglobin, Der Sauerstoffträger kann aber natürlich in der Nanoemulsion auch ohne die Sauerstoffbeladung vorliegen.

Der im erfindungsgemässen Verfahren verwendete amphotere Emulgator, der vorzugsweise ein biologischer oder biologisch hergestellter Emulgator sein kann, kann zweckmässig ein Glycerinphosphatid der Formel

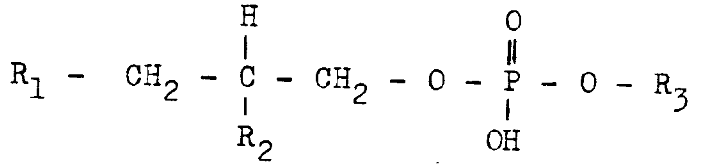

(I)

sein, in welcher $R_1$ und $R_2$ Acyloxy und/oder Alkyl- oder Alkenyläther sind und $R_3$ Triniederalkylammonio, z.B. Trimethylammonio, oder aminosubstituiertes Niederalkyl darstellen, z.B. 2-Trimethylammonioäthyl (Cholinyl). $R_1$ und $R_2$ sind vorzugsweise Acyloxy, d.h. esterverknüpfte Fettsäurereste; Alkyl- oder Alkenyläther, d.h. ätherverknüpfte Fettsäurereste, sind jedoch ebenfalls möglich. $R_3$ ist vorzugsweise 2-Trimethylammonioäthyl oder 2-Aminoäthyl Ein Glycerinphosphatid der Formel (I), in der $R_1$, $R_2$ und $R_3$ die zuletzt angegebenen Bedeutungen haben, ist z.B. ein natürliches Lecithin, beispielsweise Ei-Lecithin oder Lecithin aus Sojabohnen ($R_3$ = 2-Trimethylammonioäthyl), oder ein natürliches Kephalin, z.B. Ei-Kephalin oder Kephalin aus Sojabohnen ($R_3$ = 2-Aminoäthyl).

Der Emulgator soll in der wässrigen Phase eine möglichst weitgehend ideale lamellare flüssigkristalline Struktur aufweisen. Diese bildet die Grenzschicht zwischen der äusseren wässrigen Phase und der inneren Oelphase. Die physikalischen Bedingungen für eine stabile Emulsion (solche Emulsionen zeigen keine Koaleszenz) sind: maximale spezifische Grenzfläche und maximale Grenzflächenenergie der Partikel, sowie minimale Grenzflächenspannung, was praktisch bedeutet, dass möglichst kleine Partikel, z.B. 50 bis 100 nm, mit homogener Grössenverteilung hergestellt werden sollten. In der nach dem erfindungsgemässen Verfahren hergestellten Nanoemulsion wird Koaleszenz verhindert durch die Einhaltung des angegebenen Massenverhältnisses Emulgator/Oel von 0,05 bis 0,4, vorzugsweise 0,1 bis 0,35. Zusätzlich sollte die Partikeldichte, d.h. die Gesamtlipidkonzentration (Emulgator + Oel), vorzugsweise höchstens 20 Gew.% betragen, bei Partikelgrössen von 50 bis 100 nm. Ferner sollte zweckmässig auf eine intensive Interaktion der Moleküle der lamellaren Emulgatorstruktur mit denjenigen der Oelphase geachtet werden.

Zusätzliche bevorzugte Glycerinphosphatide (als Emulgatoren) sind auch synthetische Lecithine ($R_3$ = 2-Trimethylammonioäthyl) und synthetische Kephaline ($R_3$ = 2-Aminoäthyl) der Formel (I), worin $R_1$ und $R_2$ identische Acyloxyreste, z.B. Lauroyloxy, Oleoyloxy, Linoyloxy, Linoleoyloxy oder Arachinoyloxy, bedeuten, z.B. Dilauroyl-, Dimyristoyl-, Dipalmitoyl-, Distearoyl-, Dioleoyl-, Dilinoyl-, Dilinoleoyl- oder Diarachinoyllecithin oder -kephalin, oder $R_1$ und $R_2$ verschiedene Acyloxyreste bedeuten, z.B. $R_1$ Palmitoyloxy und $R_2$ Oleoyloxy, z.B. 1-Palmitoyl-2-oleoyl-lecithin oder -kephalin, oder $R_1$ und $R_2$ identische Alkoxyreste bedeuten, z.B. Tetradecyloxy oder Hexadecyloxy, z.B. Ditetradecyl- oder Dihexadecyllecithin oder -kephalin, $R_1$ Alkenyl und $R_2$ Acyloxy, z.B. ein Plasmalogen ($R_3$ = Trimethylammonioäthyl), oder $R_1$ Acyloxy, z.B. Myristoyloxy oder Palmitoyloxy, und $R_2$ Hydroxy, z.B. natürliches oder synthetisches Lysolecithin oder Lysokephalin, z.B. 1-Myristoyl- oder 1-Palmitoyllysolecithin oder -kephalin, darstellen. Wie schon erwähnt, kann dabei an die Stelle der Esterverknüpfung auch eine Aetherverknüpfung treten.

Ein geeignetes Lipid ist ferner ein Lipid der Formel (I), worin $R_1$ einen Alkenylrest, $R_2$ einen Acylamidorest und $R_3$ 2-Trimethylammonioäthyl (Cholinrest) darstellen. Ein solches Lipid ist unter dem Namen Sphingomyelin bekannt.

Ein geeignetes Lipid ist ausserdem ein Lysolecithin-Analoges, z.B. 1-Lauroyl-1,3-propandiol-3-phosphorylcholin, ein Monoglycerid, z.B. Monoolein oder Monomyristin, ein Cerebrosid, ein Gangliosid oder ein Glycerid, welches keine freie oder veresterte Phosphoryl- oder Phosphonylgruppe in 3-Stellung enthält. Ein solches Glycerid ist beispielsweise ein Diacylglycerid oder 1-Alkenyl-1-hydroxy-2-acylglycerid mit den genannten Acyl- bzw. Alkenylgruppen, worin die 3-Hydroxygruppe durch einen der genannten Kohlenhydratreste, z.B. einen Galactosylrest, veräthert ist, z.B. ein Monogalactosylglycerin.

Ein anderes geeignetes Lipid ist ferner ein neutrales Lipid, welches in Zellmembranen enthalten und nur in apolaren organischen Lösungsmitteln, z.B. in Chloroform, löslich ist. Neutrale Lipide sind beispielsweise Steroide, z.B. Oestradiol oder Sterine, z.B. Cholesterin, β-Sitosterin, Desmosterin, 7-Keto-Cholesterin oder β-Cholestanol, fettlösliche Vitamine, z.B. Vitamin A, z.B. Vitamin $A_1$ oder $A_2$, Vitamin E, Vitamin K, z.B. Vitamin $K_1$ oder $K_2$ oder Vitamin $D_2$ oder $D_3$.

Das im erfindungsgemässen Verfahren verwendete Oel kann ein flüssiges Fett tierischer, pflanzlicher oder synthetischer Herkunft mit der folgenden allgemeinen Struktur sein:

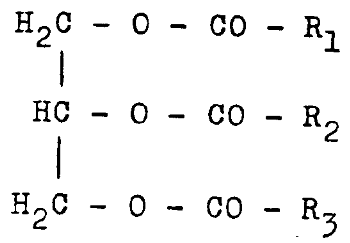

( II )

$R_1$, $R_2$ und $R_3$ stellen gesättigte und ungesättigte Fettsäurereste (unverzweigt und verzweigt) dar mit variablen Kettenlängen bis maximal $C_{24}$ einschliesslich halbsynthetische Derivate. Die gebräuchlichsten pharmazeutischen Triglyceride sind: Erdnussöl, Sojabohnenöl, Baumwollsamenöl und Miglyole®.

Ferner kann die Oelphase aus Fettsäureestern bestehen. Als Fettsäurekomponenten kommen dieselben Fettsäuren in Betracht, wie sie bei den Triglyceriden beschrieben wurden. Die Fettsäureester enthalten Alkoholreste mit Kohlenstoffketten von $C_1$ bis $C_{24}$. Die Alkohole können unverzweigte, verzweigte sowie gesättigte und ungesättigte Strukturen aufweisen. Synthetische Derivate von ein- und mehrwertigen Alkoholen sind ebenfalls eingeschlossen. Gebräuchliche pharmazeutische Fettsäureester sind: Palmitinsäureisopropylester, Myristinsäureisopropylester, Oelsäureethylester.

Für die Herstellung einer Nanoemulsion kann allgemein wie folgt vorgegangen werden:

Eine O/W-Voremulsion wird erreicht durch einfaches Schütteln des Emulgator/Oel-Wassergemisches während einiger Minuten, bis optisch beurteilt die gesamte Oelmenge eingearbeitet ist. Die Temperatur ist dabei so zu wählen, dass oberhalb der Uebergangstemperatur des Emulgators (vorliegend der flüssig-kristallinen Struktur) gearbeitet wird.

Die erhaltene O/W-Voremulsion wird in einen Rochdruckhomogenisator handelsüblicher Herkunft transferiert und dort auf die gewünschte Partikelgrösse verkleinert. Dabei wird die Temperatur konstant gehalten und liegt wiederum oberhalb der Uebergangstemperatur des gewählten Emulgators.

Stabile Nanoemulsionen mit definierter, homogener Partikelgrössen-Verteilung werden erreicht durch die richtige Wahl von Emulgierzeit und -druck. Bevorzugt wird ein Kammerdruck von 500 bis 1'000 bar eingesetzt, dessen Wirkungsgrad durch die Kammergeometrie des eingesetzten Hochdruckhomogenisators beeinflusst wird.

Die resultierenden Nanoemulsionen können antimikrobiell behandelt werden durch Dampfdrucksterilisation (1 bar Ueberdruck, 20 min. bei 120°C) oder Keimfiltration (0,2 $\mu$m Porengrösse).

Zur Charakterisierung der hergestellten Nanoemulsionen werden eingesetzt:

- optische Beurteilung: schwache bis starke Opaleszenz des Präparats ist leicht erkennbar (Hinweis auf Partikelgrösse kleiner 200 nm)
- Laser-Streulichtmessung (Partikelgrösse und -homogenität)
- Elektronenmikroskopie (Gefrierbruch- und Negativkontrasttechnik: Verifizieren der Laser-Streulichtmessungen und Aufschluss über die Morphologie der Partikel)
- chemische Analyse der eingesetzten Nanoemulsions-Komponenten.

Beispiel 1: Herstellung einer 1-2%igen Nanoemulsion mit einem Triglycerid

250 mg des amphoteren Emulgators Lecithin (fettfrei) und 1'000 mg Sojabohnenöl als Oelkomponente werden in einen Rundkolben eingewogen. Durch Zugabe von Ethanol (ca. 15 ml) werden die beiden Substanzen gelöst, und anschliessend wird das Lösungsmittel am Rotationsverdampfer wieder entfernt. Dem Rückstand wird physiologische Kochsalzlösung ad 100 ml zugefügt. Anschliessend wird das Gemisch bis zur vollständigen Einarbeitung des Oels (optisch beurteilt) geschüttelt.

Diese Voremulsion wird auf den Hochdruckhomogenisator handelsüblicher Herkunft übertragen und auf die gewünschte Partikelgrösse von ca. 100 nm verkleinert. Dabei wird mit einem Kammerdruck von 950 bar während 20 min. homogenisiert. Dieser Prozess wird bei konstanter Temperatur von 35°C durchgeführt. Diese Nanoemulsion ist über mehrere Monate stabil. Das Produkt ist in nachstehender Tabelle 1 charakterisiert.

Tabelle 1

1. optisch: leicht milchig, opaleszent, im Gegenlicht transparent

2. chemisch:

| Komponente | Konzentration (mg/ml) in der physiologischen Kochsalzlösung | |
|---|---|---|
| | Einwaage | gemessen in Nanoemulsion |
| Lecithin | 2,5 | 2,53 ± 0,07[1)] |
| Sojabohnenöl | 10,0 | 9,83 ± 0,22[2)] |

1) Hochdruckflüssigchromatographie (HPLC) und enzymatische Bestimmung

2) Enzymatische Bestimmung

3. Laser-Streulichtmessung:
Mittlerer Partikeldurchmesser in nm der Nanoemulsion, mittels Gauss-Analyse bestimmt. 100 ± 30

Beispiel 2: Herstellung einer 5-6%igen Nanoemulsion mit einem Fettsäureester

2,8 g des amphoteren Emulgators Lecithin (fettfrei) in Form von unilamellaren Liposomen definierter Vesikelgrösse (55 nm) in wässriger Phase und 12 g Myristinsäureisopropylester werden in einem Rundkolben vorgelegt. Diese wässrige Phase mit einem Volumen von 250 ml wird bis zur vollständigen Einarbeitung des Oels (optisch beurteilt) von Hand geschüttelt. Diese Voremulsion wird auf den Hochdruckhomogenisator handelsüblicher Herkunft übertragen und auf die gewünschte Partikelgrösse von ca. 70 nm verkleinert. Dabei wird mit einem Kammerdruck von 950 bar während 20 min. homogenisiert. Dieser Prozess wird bei konstanter Temperatur von 35°C durchgeführt. Diese Nanoemulsion ist über mehrere Monate stabil. Das Produkt ist in nachstehender Tabelle 2 charakterisiert:

Tabelle 2

1. optisch: milchig, opaleszent, im Gegenlicht transparent

2. chemisch;

| Komponente | Konzentration (mg/ml) in der physiologischen Kochsalzlösung | |
|---|---|---|
| | Einwaage | gemessen in Nanoemulsion |
| Lecithin | 11,2 | 11,3 ± 0,3[1)] |
| Myristinsäure-isopropylester | 48,0 | 45,8 ± 2,3[2)] |

1) Hochdruckflüssigchromatographie (HPLC) und enzymatische Bestimmung

2) quantitative Dünnschichtchromatographie (HPTLC)

3. Laser-Streulichtmessung:

| | |
|---|---|
| Mittlerer Partikeldurchmesser in nm der Nanoemulsion, mittels Gauss-Analyse bestimmt | 66 ± 27 |

Nach dem beschriebenen Verfahren hergestellte Nanoemulsionen können direkt als Parenteralien zur Fetternährung verwendet werden.

Ferner kann man den Nanoemulsionen auch Proteine und/oder APO-Proteine zusetzen, um Lipoprotein-Emulsionen zu bilden. Wegen der kleinen Partikelgrösse verhalten sich die Oel-Emulgator-Partikel physiologisch analog zu den normalerweise im Blut gebildeten Chylomikronen-Remnants, die von der Leber, d.h. den Hepatocyten, hochspezifisch aufgenommen werden.

Die beschriebenen Nanoemulsionen verhalten sich physiologisch wie die "Low-Density-Lipoproteine" und hemmen analog in den Hepatocyten die Cholesterin-Biosynthese.

Die mit dem erfindungsgemässen Verfahren hergestellten Oel-Emulgator-Partikel bzw. die künstlich hergestellten Lipoproteine eignen sich auch speziell als Transportvehikel für Pharmaka, die gezielt in den Hepatocyten wirken.

Die Nanoemulsionen können aber auch als Träger für andere Wirkstoffe dienen, z.B. für andere Arzneistoffe und/oder kosmetische Ingredienzien. Solche können der fertigen Nanoemulsion zugesetzt werden, oder auch vor der Herstellung der Nanoemulsion zur wässrigen Phase oder zum Oel oder zum Emulgator gegeben werden, und sie werden dann an die Oelpartikel und/oder an den Emulgator angelagert und/oder in diese eingebaut bzw. in denselben gelöst.

Nanoemulsionen, welche pharmazeutische Wirkstoffe enthalten, können für die Herstellung von pharmazeutischen Präparationen verwendet werden, wobei die Nanoemulsion als aktiver Bestandteil mit einem zur therapeutischen Verabreichung geeigneten, festen oder flüssigen Träger vermischt wird. Dem Gemisch kann man gegebenenfalls eine besondere galenische Form geben. Folgende galenische Darreichungsformen können dabei in Betracht gezogen werden:

- Ampullen, insbesondere sterile Injektions- und Infusionslösungen;
- Lösungen, insbesondere Sirupe, Augen- und Nasentropfen, die ausser der Nanoemulsion verschiedene Hilfsstoffe enthalten können;

- nicht dosierende Aerosole und Dosier-Aerosole, die ausser der Nanoemulsion Treibgas und Stabilisatoren enthalten können;
- hydrophile und hydrophobe Gele und Salben, welche die Nanoemulsion enthalten;
- O/W- oder W/O-Cremen, welche die Nanoemulsion enthalten;
- Lotionen und Pasten, welche die Nanoemulsion enthalten.

Nach dem beschriebenen Verfahren hergestellte Nanoemulsionen können auch vorteilhaft für die Herstellung von Nährstofflösungen für Zellkulturen verwendet werden, indem den Nanoemulsionen z.B. natürliche Aminosäuren, Antibiotika, geringe Mengen Transferrin und gewünschtenfalls Glucose zugesetzt werden. In solchen Nährstofflösungen dienen die Nanoemulsionen als Energielieferanten und können die in bekannten Nährstofflösungen verwendeten Proteine, z.B. aus Kälberserum, mindestens zum Teil ersetzen.

## Patentansprüche

1. Verfahren zur Herstellung einer Nanoemulsion von weniger als 200 nm grossen Oelpartikeln aus Triglycerid oder Fettsäureester in einer wässrigen Phase mittels eines Hochdruckhomogenisators, dadurch gekennzeichnet, dass die wässrige Phase, das Oel und pro Gewichtsteil Oel 0,1 bis 0,4 Gewichtsteile eines amphoteren Glycerinphosphatid-Emulgators so zusammengemischt werden, dass der Emulgator in der wässrigen Phase eine Doppelschichtstruktur bildet, und dass die Mischung dann in dem Hochdruckhomogenisator zur Nanoemulsion verarbeitet wird.

2. Verfahren nach Anspruch 1, dadurch gekennzeichnet, dass die Gesamtkonzentration von Oel und Emulgator in der wässrigen Phase höchstens 20 Gew.% beträgt.

3. Verfahren nach Anspruch 1 oder 2, dadurch gekennzeichnet, dass als Emulgator ein Glycerinphosphatid der Formel

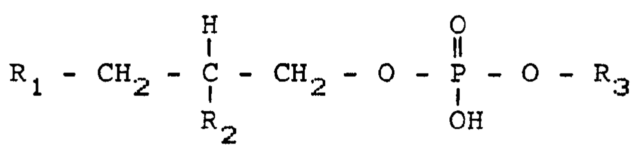

(I)

verwendet wird, in welcher $R_1$ und $R_2$ Acyloxy und/oder Alkyl- oder Alkenyläther sind und $R_3$ Triniederalkylammonio oder aminosubstituiertes Niederalkyl ist.

4. Verfahren nach Anspruch 3, dadurch gekennzeichnet, dass als Emulgator Lecithin verwendet wird.

5. Verfahren nach einem der Ansprüche 1 bis 4, dadurch gekennzeichnet, dass die Nanoemulsion bei einer Temperatur oberhalb der Uebergangstemperatur des Emulgators hergestellt wird.

6. Verfahren nach einem der Ansprüche 1 bis 5, dadurch gekennzeichnet, dass zu der Nanoemulsion nach der Verarbeitung im Hochdruckhomogenisator Protein oder APO-Protein zugesetzt wird, welches sich an die Oelpartikel anlagert, so dass eine Lipoproteinemulsion erhalten wird.

7. Verfahren nach einem der Ansprüche 1 bis 6, dadurch gekennzeichnet, dass die Nanoemulsion nach der Verarbeitung im Hochdruckhomogenisator durch Dampfdrucksterilisation oder Keimfiltration antimikrobiell behandelt wird.

8. Verfahren nach einem der Ansprüche 1 bis 7, dadurch gekennzeichnet, dass den Ausgangssubstanzen oder dem Gemisch derselben oder der Nanoemulslon nach der Verarbeitung im Hochdruckhomogenisator ein Wirkstoff oder ein Sauerstoffträger zugesetzt wird.

9. Verwendung der nach dem Verfahren gemäss Anspruch 8 hergestellten Nanoemulsion für die Herstellung pharmazeutischer und/oder kosmetischer Präparationen, wobei die Nanoemulsion als aktiver Bestandteil mit einem zur therapeutischen Verabreichung geeigneten, inerten, festen oder flüssigen Träger vermischt wird und dem Gemisch gegebenenfalls eine besondere galenische Form gegeben wird.

10. Verwendung der nach dem Verfahren gemäss einem der Ansprüche 1 bis 7 hergestellten Nanoemulsion

für die Herstellung einer Nährstofflösung für Zellkulturen, in der die Nanoemulsion als Energlelieferant dient.

## Claims

1. Process for the production of a nanoemulsion of oil particles less than 200 nm in size of a triglyceride or fatty acid ester in an aqueous phase by means of a high-pressure homogenizer, characterized in that the aqueous phase, the oil and, per part by weight of oil, 0.1 - 0.4 part by weight of an amphoteric glycerophosphatide emulsifier are mixed together in such a manner that the emulsifier forms a bilayer structure in the aqueous phase, and in that the mixture is then processed to the nanoemulsion in the high-pressure homogenizer.

2. Process according to claim 1, characterized in that the total concentration of oil and emulsifier in the aqueous phase is at most 20% by weight.

3. Process according to claim 1 or 2, characterized by using as the emulsifier a glycerophosphatide of the formula

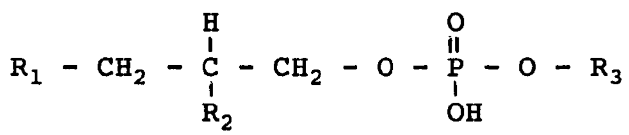

(I)

wherein $R_1$ and $R_2$ mean acyloxy and/or alkyl or alkenyl ether, and $R_3$ is tri-lower-alkyl ammonio or amino-substituted lower alkyl.

4. Process according to claim 3, characterized by using lecithin as the emulsifier.

5. Process according to any one of claims 1 to 4, characterized by preparing the nanoemulsion at a temperature above the transition temperature of the emulsifier.

6. Process according to any one of claims 1 to 5, characterized by adding proteins or apoproteins to the nanoemulsion after processing in the high-pressure homogenizer, these proteins or apoproteins then attaching themselves to the oil particles so that a lipoprotein emulsion is obtained.

7. Process according to any one of claims 1 to 6, characterized in that the nanoemulsion, after processing in the high-pressure homogenizer, is treated anti-microbially by pressurized steam sterilization or germ filtration.

8. Process according to any one of claims 1 to 7, characterized in that an active agent or an oxygen carrier is added to the starting materials or to the mixture thereof or to the nanoemulsion after processing in the high-pressure homogenizer.

9. Use of the nanoemulsion produced according to the process of claim 8, for the production of pharmaceutical and/or cosmetic preparations wherein the nanoemulsion is mixed, as the active component, with an inert, solid or liquid vehicle suitable for therapeutic administration, and optionally a special galenic form is imparted to the mixture.

10. Use of the nanoemulsion produced according to the process of any one of claims 1 to 7, for the production of a nutrient solution for cell cultures wherein the nanoemulsion serves as the energy supplier.

## Revendications

1. Procédé pour préparer une nano-émulsion de particules huileuses d'une grosseur inférieure à 200 nm de triglycéride ou d'un ester d'acides gras dans une phase aqueuse, à l'aide d'un homogénéisateur fonctionnant sous pression élevée, procédé caractérisé en ce qu'on mélange la phase aqueuse, l'huile et, par partie en poids d'huile 0,1 à 0,4 partie en poids d'un émulsifiant, qui est du glycérophosphatide amphotère, de façon que l'émulsifiant forme en phase aqueuse une structure à couche double, et en ce qu'on traite ensuite le mélange dans l'homogénéisateur fonctionnant sous pression élevée pour transformer ce mélange en une nano-émulsion.

**2.** Procédé selon la revendication 1, caractérisé en ce que la concentration totale de l'huile et de l'émulsifiant dans la phase aqueuse vaut au maximum 20 % en poids.

**3.** Procédé selon la revendication 1 ou 2, caractérisé en ce qu'on utilise comme émulsifiant un glycérophosphatide de formule

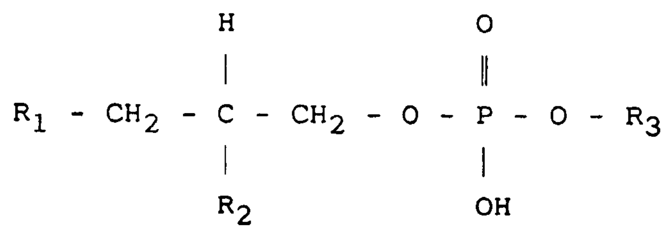

(I)

dans laquelle $R_1$ et $R_2$ représentent chacun un groupe acyloxy et/ou éther d'alkyle ou d'alcényle (alkyloxy ou alcényloxy), et $R_3$ représente un groupe alkyle inférieur à substitution tri(alkyl inférieur) ammonio ou amino.

**4.** Procédé selon la revendication 3, caractérisé en ce qu'on utilise la lécithine comme émulsifiant.

**5.** Procédé selon l'une des revendications 1 à 4, caractérisé en. ce qu'on prépare la nano-émulsion à une température supérieure à la température de transition de l'émulsifiant.

**6.** Procédé selon l'une des revendications 1 à 5, caractérisé en ce qu'on ajoute à la nano-émulsion, après son traitement de transformation dans un homogénéisateur fonctionnant sous pression élevée, de la protéine ou de l'apoprotéine, qui se fixe sur les particules d'huile, de sorte que l'on obtient une émulsion de lipoprotéines.

**7.** Procédé selon l'une des revendications 1 à 6, caractérisé en ce que, après son traitement de transformation dans un homogénéisateur fonctionnant sous pression élevée, on applique à la nano-émulsion un traitement antimicrobien effectué par stérilisation à la vapeur d'eau sous pression ou par filtration pour élimination des germes.

**8.** Procédé selon l'une des revendications 1 à 7, caractérisé en ce qu'on ajoute une substance active ou un porteur d'oxygène aux substances de départ ou à leur mélange ou à la nano-émulsion après son traitement de transformation dans un homogénéisateur fonctionnant sous pression élevée.

**9.** Utilisation de la nano-émulsion, préparée selon le procédé de la revendication 8, pour la confection de préparations pharmaceutiques et/ou cosmétiques, la nanoémulsion , à titre de constituant actif, étant mélangée à un véhicule ou support inerte, solide ou liquide, convenant pour une administration thérapeutique et le mélange étant éventuellement mis sous une forme galénique particulière.

**10.** Utilisation de la nano-émulsion, produite par le procédé selon l'une des revendications 1 à 7, pour la confection d'une solution nutritive pour des cultures de cellules, dans laquelle la nano-émulsion sert d'agent fournisseur d'énergie.